(19) Europäisches Patentamt
European Patent Office
Office européen des brevets

(11) Publication number: **0 004 122 B2**

(12) **NEW EUROPEAN PATENT SPECIFICATION**

(45) Date of publication of the new patent specification: **12.06.85**

(51) Int. Cl.[4]: **C 07 C 33/30**, C 07 C 29/14, C 07 C 43/20, C 07 C 41/00

(21) Application number: **79200121.6**

(22) Date of filing: **10.03.79**

(54) **Process for the preparation of alpha,beta-unsaturated alcohols.**

(30) Priority: **13.03.78 NL 7802694**

(43) Date of publication of application: **19.09.79 Bulletin 79/19**

(45) Publication of the grant of the patent: **12.06.85 Bulletin 85/24**

(45) Mention of the opposition decision: **30.09.81 Bulletin 81/39**

(84) Designated Contracting States: **BE CH DE FR GB IT NL**

(56) References cited:
**DE-C-1 568 861**
**FR-A-1 489 504**
**GB-A-1 086 447**
**US-A-4 073 813**

**Izvestiya Akad. Nauk, SSSR Seriya Khimicheskaya, No. 1, pages 77-82, 1969**

(73) Proprietor: **STAMICARBON B.V.**
**Postbus 10**
**NL-6160 MC Geleen (NL)**

(72) Inventor: **Dautzenberg, Jozef Marie Andreas**
**Laathofstraat 21**
**NL-6191 GD Beek (L) (NL)**
Inventor: **Mulders, Joannes Maria Cornelis Anthonius**
**v. Goudoeverstraat 20**
**NL-6166 XC Geleen (NL)**
Inventor: **Stijfs, Petrus Antonius Maria Juliana**
**Schoutenstraat 36**
**NL-6151 GS Munstergeleen (NL)**

(74) Representative: **Hoogstraten, Willem Cornelis Roeland et al**
**OCTROOIBUREAU DSM Postbus 9**
**NL-6160 MA Geleen (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 004 122 B2

**Description**

The invention relates to a process for preparing α-β unsaturated alcohols by catalytic hydrogenation in the liquid phase of the aldehyde corresponding to the desired alcohol. Alcohols of this type are of practical importance as starting materials in the flavour and fragrance industry.

When an α-β unsaturated aldehyde is hydrogenated to prepare the corresponding alcohol, the hydrogenation should be effected so that substantially no hydrogenation of the double bond occurs. To achieve this, various catalysts have been proposed for this hydrogenation. The catalyst recommended in United States Patent Specification 3,284,517 is platinum on carbon with divalent iron and silver as promoters. According to British Patent Specification 1,123,837 platinum with an alkali metal hydroxide or alkali metal alkoxide as promoter is very suitable as a catalyst. United States Patent Specification 3,655,777 states that platinum is unsuitable as a catalyst, but that very good results can be obtained with osmium. British Patent Specification 1,439,711 describes the successful use of platinum-cobalt catalyst.

When these known catalysts are used for the selective hyrogenation of α-β unsaturated aldehydes, the amount of catalyst required is rather large and the conversion of the aldehyde is often incomplete, so that the known methods are little attractive economically.

The invention provides for such hydrogenation in which substantially complete conversion can be achieved with a considerably lower catalyst consumption.

The process according to the invention for preparing α-β unsaturated alcohols by liquid phase hydrogenation, with a platinum containing catalyst and alkali metal hydroxide and/or an alkali metal alkoxide as promoter, of the aldehyde corresponding to the desired alcohol is characterized in that the starting aldehyde has the general formula

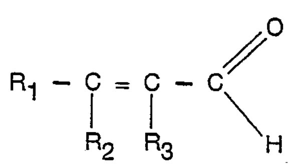

in which $R_1$, $R_2$ and $R_3$, which may be the same or different, represent phenyl, optionally substituted with one or more alkyl and/or alkoxy groups in which substituents the total number of C-atoms is at most 5, or hydrogen, or an alkyl group with 1—10 C-atoms is at most 5, or hydrogen, or an alkyl group with 1—10 C-atoms, and at least one of the groups $R_1$, $R_2$ and $R_3$ represents said phenyl or substituted phenyl group, and in that the hydrogenation is effected in a reaction medium comprising water and a water-immiscible organic solvent.

Examples of aldehydes that are very suitable for use in the process according to the invention are 3-phenyl-2-propenal (cinnamic aldehyde), 2-n-pentyl-3-phenyl-2-propenal, 2-n-hexyl-3-phenyl-2-propenal, 2-methyl-3-phenyl-2-propenal, 2-phenyl-2-pentenal, 2-phenyl-2-propenal and 3-p-methoxyphenyl-2-propenal.

In the process according to the invention various water-immiscible organic solvents may be used, e.g., benzene, xylones, cyclopentane, cyclohexane, n-pentane and n-hexane. Use is preferably made of toluene. The ratio between the water and the organic solvent may be varied between 0.05 and 3 grams of water per gram of organic solvent. The use of 0.2—1 gram of water per gram of organic solvent is particularly suitable. The amount of organic solvent per gram of aldehyde to be converted may be chosen with wide limits, e.g. between 0.25 and 20 grams, preferably between 0.5 and 5 grams of organic solvent per gram of aldehyde to be converted.

The catalyst may be any of the platinum catalysts that are well-known in hydrogenation, e.g. in an amount corresponding to 0.03—5 milligrams, preferably 0.05—2.5 milligrams, of platinum per gram of aldehyde to be converted.

The amount of alkali metal hydroxide and/or alkali metal alkoxide dissolved in the water amounts to, e.g. 0.1—0.7 mole, preferably 0.2—0.5 mole, per 100 grams of water.

In the hydrogenation according to the invention the temperature is so chosen that it is not too high. Very suitable are temperatures of below 60°C, preferably of between −5 and 45°C. Naturally the temperature will not be lower than that at which solids start crystallizing from the liquid phase.

The hydrogen pressure as such is not critical. Good results are usually obtained with the use of a partial hydrogen pressure of between 500 and 30,000 kPa.

Upon completion of the hydrogenation in the process according to the invention, the liquid reaction medium is separated into an organic layer containing the desired reaction product and into an aqueous layer containing the catalyst and the promoter used. This aqueous layer is re-used in the hydrogenation of the aldehyde. The unsaturated alcohol may be recovered from the organic layer, e.g. by distillation, while the organic solvent may be re-used.

The process according to the invention will be further elucidated in the following examples.

Examples I—XV

An amount of water in which alkali metal hydroxide or alkali metal alkoxide has been dissolved as a promoter is put into a 0.5-litre autoclave provided with a stirrer. An amount of commercially available platinum catalyst and a solution of the aldehyde to be hydrogenated in the organic solvent are then added to this alkaline solution. The autoclave is then closed, after which the hydrogenation is effected and the reaction conditions are maintained until no more hydrogen is absorbed.

| Example No. | aldehyde | temp. °C | hydrogen pressure kPa | catalyst g | promoter | water g | reaction time h | conversion aldehyde % | Yields in % | | |
|---|---|---|---|---|---|---|---|---|---|---|---|
| | | | | | | | | | α–β unsaturated alcohol | saturated alcohol | saturated aldehyde |
| I | 3-phenyl-2-propenal | 20 | 12000 | 0.18 | 10.2 g KOH | 60 | 6 | 99.4 | 92.4 | 5.2 | 0.8 |
| II | 2-methyl-3-phenyl-2-propenal | 20 | 12000 | 0.18 | 10.2 g KOH | 60 | 6 | 99.7 | 93.6 | 4.3 | 0.3 |
| III | 2-hexyl-3-phenyl-2-propenal | 20 | 12000 | 0.18 | 10.2 g KOH | 60 | 7 | 99.2 | 94.1 | 4.6 | 0.5 |
| IV | 2-amyl-3-phenyl-2-propenal | 20 | 12000 | 0.18 | 10.2 g KOH | 60 | 7 | 99.6 | 93.2 | 4.8 | 0.6 |
| V | 3-phenyl-2-propenal | 20 | 12000 | 0.18 | 10.2 g KOH | 60 | 6 | 99.1 | 91.6 | 5.6 | 1.0 |
| VI | 3-phenyl-2-propenal | 20 | 12000 | 0.18 | 10 g $CH_3ONa$ | 60 | 6 | 99.2 | 92.0 | 5.3 | 0.9 |
| VII | 3-phenyl-2-propenal | 20 | 12000 | 0.09 | 10.2 g KOH | 60 | 7 | 98.7 | 91.1 | 5.8 | 1.2 |
| VIII | 3-phenyl-2-propenal | 40 | 12000 | 0.18 | 10.2 g KOH | 60 | 4 | 98.9 | 83.1 | 8.3 | 1.4 |
| IX | 3-phenyl-2-propenal | 20 | 12000 | 0.25 | 10.2 g KOH | 60 | 3 | 98.3 | 91.6 | 5.1 | 1.8 |
| X | 3-phenyl-2-propenal | 4 | 12000 | 0.50 | 10.2 g KOH | 60 | 4 | 98.7 | 93.3 | 3.7 | 0.4 |
| XI | 3-phenyl-2-propenal | 20 | 20000 | 0.18 | 10.2 g KOH | 60 | 3.5 | 97.1 | 94.1 | 3.6 | 2.0 |
| XII | 3-phenyl-2-propenal | 0 | 2000 | 3 | 6 g KOH | 40 | 4 | 97.8 | 93.4 | 5.2 | 0.4 |
| XIII | 3-phenyl-2-propenal | 37 | 2000 | 1 | 5.1 g KOH | 30 | 2.5 | 99.6 | 86.0 | 10.2 | 0.7 |
| XIV | 2-methyl-3-phenyl-2-propenal | 0 | 2000 | 3 | 6 g KOH | 40 | 4 | 98.7 | 94.1 | 4.8 | 0.2 |
| XV | 3-phenyl-2-propenal | 0 | 2000 | 2 | 5.1 g KOH | 40 | 4 | 99.5 | 89.2 | 6.5 | 0.2 |

**Claim**

1. Process for preparing α-β unsaturated alcohols by liquid phase hydrogenation, with a platinum containing catalyst and an alkali metal hydroxide and/or alkali metal alkoxide as promoter, of the aldehyde corresponding to the desired alcohol, characterized in that the starting aldehyde has the general formula

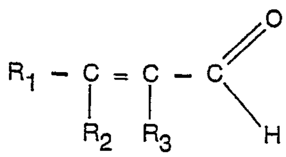

in which $R_1$, $R_2$ and $R_3$, which may be the same or different, represent phenyl, phenyl substituted with one or more alkyl and/or alkoxy groups in which substituents the total number of C-atoms is at most 5, or hydrogen or an alkyl group with 1-10 C-atoms, and at least one of the groups $R_1$, $R_2$ and $R_3$ represent said phenyl or substituted phenyl group, and in that the hydrogenation is effected in a reaction medium comprising a water-immiscible organic solvent and water in an amount of between 0,05 and 3 gram of water per gram of organic solvent, and that after completion of the reaction the reaction medium is separated in an aqueous phase containing the catalyst and promoter, which aqueous phase is re-used, and an organic phase containing the reaction products.

2. Process according to claim 1, characterized in that the water-immiscible organic solvent is toluene.

3. Process according to any one of claims 1—2, characterized in that 0.2—1 gram of water is used per gram of organic solvent.

4. Process according to any one of the claims 1—3, characterized in that 0.5—5 grams of the organic solvent are used per gram of aldehyde to be converted.

5. Process according to any one of the claims 1—4, characterized in that the hydrogenation is effected by means of a catalyst containing platinum in an amount of 0.05—2.5 milligram per gram of aldehyde to be hydrogenated.

6. Process according to any one of the claims 1—5, characterized in that the dissolved alkali metal hydroxide and/or alkali metal alkoxide is used in an amount of 0.2—0.5 mole per 100 grams of water.

7. Process according to any one of the claims 1—6, charaterized in that the hydrogenation is effected at a temperature of between −5 and 45°C.

8. Process according to any one of claims 1—7, characterized in that the hydrogenation is effected at a partial hydrogen pressure of between 500 and 30,000 kPa.

9. Process according to any one of the claims 1—8, characterized in that the starting material is 3-phenyl-2-propenal, 2-n-pentyl-3-phenyl-2-propenal, 2-n-hexyl-3-phenyl-2-propenal, 2-methyl-3-phenyl-2-propenal, 2-phenyl-2-pentenal, 2-phenyl-2-propenal or 3-p-methoxyphenyl-2-propenal.

**Revendications**

1. Procédé de préparation d'α-β alcools non saturés au moyen d'une hydrogénation catalytique en phase liquide — en utilisant un catalyseur contenant du platine et un hydroxyde de métal alcalin et/ou un alcoxyde de métal alcalin comme promoteur — de l'aldéhyde correspondant à l'alcool désiré, caracterisé en ce que l'aldéhyde de départ a la formule générale

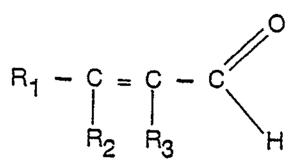

dans laquelle $R_1$, $R_2$, et $R_3$, qui peuvent être semblables ou différents, représentent du phényl, du phényl substitué avec un ou plusieurs groupes alkyl et/ou alcoxy, dans lesquels substituants le nombre total des atomes de C est de 5 au maximum, ou de l'hydrogène ou un groupe alkyl avec 1—10 atomes de C, alors que du moins un des groupes $R_1$, $R_2$ et $R_3$ représente ledit phényl ou le groupe de phényl substitué et que l'hydrogénation est effectuée dans un milieu réactionnel comprenant un solvant organique non miscible à l'eau et de l'eau en une quantité d'entre 0,05 et 3 grammes d'eau par gramme de solvant organique, et en ce que, après l'achèvement de la réaction, le milieu réactionnel est séparé en une phase aqueuse contenant le catalyseur et le promoteur, laquelle phase aqueuse est réutilisée, et une phase organique contenant les produits de la réaction.

2. Procédé selon la revendication 1, caractérisé en ce que la solvant organique non miscible à l'eau est du toluène.

3. Procédé selon l'une des revendications 1 à 2, caractérisé en ce qu'on utilise 0.2—1 g d'eau par gramme de solvant organique.

4. Procédé selon l'une des revendications 1 à 3, caractérisé en ce qu'on utilise 0,5—5 g de solvant organique par gramme d'aldéhyde à convertir.

5. Procédé selon l'un des revendications 1 à 4, caracterisé en ce que l'hydrogénation est effectuée à l'aide d'un catalyseur contentant du platine en une quantité de 0,05—2.5 milligramme par gramme d'aldéhyde à hydrogéner.

6. Procédé selon l'une des revendications 1 à 5, caractérisé en ce qu'on utilise 0,2—0,5 mole d'hydroxyde de métal alcalin et où d'alcoxyde de métal alcalin dissous par 100 grammes d'eau.

7. Procédé selon l'une des revendications 1 à 6, caracterisé en ce que l'hydrogénation est effectuée à une température située entre −5 et 45°C.

8. Procédé selon l'une des revendications 1 à 7, caractérisé en ce que l'hydrogénation est effectuée à une pression d'hydrogéne partielle située entre 500 et 30,000 kPa.

9. Procédé selon l'une des revendications 1 à 8, caractérisé en ce qu'on utilise comme matériau de départ: 3-phényl-2-propénal, 2-n-pentyl-3-phényl-2-propénal, 2-n-hexyl-3-phényl-2-propénal, 2-methyl-3-phényl-2-propénal, 2-phényl-2-pentanal, 2-phényl-2-propénal ou 3-p-méthoxyphényl-2-propénal.

**Patentansprüche**

1. Verfahren zum Herstellen α-β-ungesättigter Alkohole durch Hydrierung in der Flüssigphase, mit einem platinhaltigen Katalysator und einem Alkalimetallhydroxyd und/oder einem Alkalimetallalkoxid als Beschleunigungsmittel, des Aldehyds entsprechend dem gewünschten Alkohol, dadurch gekennzeichnet,

dass das Ausgangsaldehyd die allgemeine Formel aufweist,

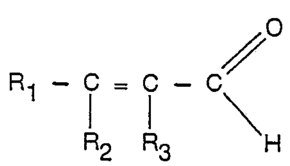

in der $R_1$, $R_2$ und $R_3$, die gleich oder verschieden sein können, Phenyl, mit einer oder mehreren Alkyl- und/oder Alkoxygruppen substituiertes Phenyl, in welchen Substituenten die Anzahl der C-Atome höchstens 5 ist, oder Wasserstoff oder eine Alkylgruppe mit 1 bis C-Atomen und mindestens eine der Gruppe $R_1$, $R_2$ und $R_3$ das Phenyl oder die substituierte Phenylgruppe darstellen,

dass die Hydrierung in einem Reaktionsmittel erfolgt, welches ein nicht wasserlösliches organisches Lösungsmittel sowie Wasser in einer Menge zwischen 0,05 und 3 Gramm Wasser je Gramm organisches Lösungsmittel enthält, und dass das Reaktionsmittel nach Beendung der Reaktion in eine wässrige Phase abgeschieden wird, die den Katalysator und das Beschleunigungsmittel enthält, welche wässrige Phase wiedergebracht wird, und eine organische Phase, die Reaktionsprodukte enthält.

2. Verfahren gemäss Anspruch 1, dadurch gekennzeichnet, dass das wasserunlösliche organische Lösungsmittel Toluol ist.

3. Verfahren gemäss Anspruch 1 oder 2, dadurch gekennziechnet, dass je Gramm organisches Lösungsmittel 0,2 bis 1 Gramm Wasser verwendet wird.

4. Verfahren gemäss einem der Ansprüche 1 bis 3, dadurch gekennzeichnet, dass je Gramm umzuwandelndes Aldehyde 0,5 bis 5 Gramm des organischen Lösungsmittel verwendet werden.

5. Verfahren gemäss einem der Ansprüche 1 bis 4, dadurch gekennzeichnet, dass die Hydrierung mit einem 0,05 bis 2,5 Milligram Platin je Gramm zu hydrierendes Aldehyd enthaltenden Katalysator durchgeführt wird.

6. Verfahren gemäss einem der Ansprüche 1 bis 5, dadurch gekennzeichnet, dass das gelöste Alkalimetallhydroxid und/oder Alkalimetallalkoxid in einer Menge von 0,2 bis 0,5 Mol je 100 Gram Wasser eingesetzt wird.

7. Verfahren gemäss einem der Ansprüche 1 bis 6, dadurch gekennzeichnet, dass die Hydrierung bei einer Temperatur zwischen −5 und 45°C erfolgt.

8. Verfahren gemäss einem der Ansprüche 1 bis 7, dadurch gekennzeichnet, dass die Hydrierung bei einem partiellen Wasserstoffdruck zwischen 500 und 30.000 kPa vorgenommen wird.

9. Verfahren gemäss einem der Ansprüche 1 bis 8, dadurch gekennzeichnet, dass das Anfangsmaterial 3-Phenyl-propenal, 2-n-Pentyl-3-phenyl-2-propenal, 2-n-Hexyl-3-phenyl-2-propenal, 2-Methyl-3-phenyl-2-propenal, 2-Phenyl-2-pentenal, 2-Phenyl-2-propenal oder 3-p-Methoxy-phenyl-2-propenal ist.